# EUROPÄISCHE PATENTANMELDUNG

(11) **EP 3 629 048 A1**
(43) Veröffentlichungstag der Anmeldung: **01.04.2020**
(21) Anmeldenummer: 18197190.4
(22) Anmeldetag: 27.09.2018
(51) Int. Cl.: G01R 33/561, G01R 33/56, G01R 33/44

(54) **NIEDERFELD-MAGNETRESONANZ-FINGERPRINTING**

(71) Anmelder: Siemens Healthcare GmbH, 91052 Erlangen (DE)
(72) Erfinder: Körzdörfer, Gregor, 91058 Erlangen (DE); Nittka, Mathias, 91083 Baiersdorf (DE); Speier, Peter, 91056 Erlangen (DE)

(57) **Zusammenfassung**

Erfindungsgemäß wird ein MRF-Verfahren zur Bestimmung von Parameterwerten in Bildpunkten eines Untersuchungsobjektes für Magnetresonanzanlagen (1) mit einer Grundmagnetfeldstärke von kleiner als 1,5 Tesla, bevorzugt mit einer Grundmagnetfeldstärke von kleiner als 0,5 Tesla vorgeschlagen. Das MRF-Verfahren kann an die bei derartigen Niederfeld-Magnetresonanzanlagen (1) herrschenden Bedingungen angepasst werden, sodass die Parameterwerte besonders effizient bei gleichzeitig hoher Qualität bestimmt werden können.

## Beschreibung

Die Erfindung betrifft ein Magnetresonanz-Fingerprinting-Verfahren für Niederfeld-Magnetresonanzanlagen zur verbesserten Bestimmung von lokalen Parameterwerten eines Untersuchungsobjektes.

Die Magnetresonanz-Technik (im Folgenden steht die Abkürzung MR für Magnetresonanz) ist eine bekannte Technik, mit der Bilder vom Inneren eines Untersuchungsobjektes erzeugt werden können. Vereinfacht ausgedrückt wird hierzu das Untersuchungsobjekt in einem Magnetresonanzgerät in einem vergleichsweise starken statischen, homogenen Grundmagnetfeld, auch B0-Feld genannt, mit Feldstärken von 0,2 Tesla bis 7 Tesla und mehr positioniert, so dass sich dessen Kernspins entlang des Grundmagnetfeldes orientieren. Zum Auslösen von Kernspinresonanzen werden hochfrequente Anregungspulse (RF-Pulse) in das Untersuchungsobjekt eingestrahlt, die ausgelösten Kernspinresonanzen als sogenannte k-Raumdaten gemessen und auf deren Basis MR-Bilder rekonstruiert oder Spektroskopiedaten ermittelt. Neben RF-Anregungspulsen können zur Manipulation der Spins in dem Untersuchungsobjekt auch weitere RF-Pulse, beispielsweise zur Refokussierung oder Inversion eines durch eine Anregung erreichten Magnetisierungszustandes, eingestrahlt werden. Dabei haben die eingestrahlten RF-Pulse nur eine Wirkung auf diejenigen Spins im Untersuchungsobjekt, deren Larmorfrequenz (Frequenz der Präzession der Spins, Resonanzfrequenz) mit der verwendeten Frequenz des RF-Pulses resonant ist ("on-resonance").

Um eine ausreichende Anzahl an Spins mit einem RF-Puls zu manipulieren, werden in der Regel RF-Pulse mit einer Mittenfrequenz und einer gewissen Frequenz-Bandbreite verwendet, die die gewünschten Larmorfrequenzen abdecken. Bei der Protonen-MR-Bildgebung wird z.B. versucht, die Larmorfrequenzen von in Wasser vorliegenden Spins anzuregen. Hat ein RF-Puls eine Wirkung auf einen Spin, so liegt dessen Larmorfrequenz in einem sogenannten Passband des RF-Pulses. Hat ein RF-Puls keine Wirkung auf einen Spin, so liegt dessen Larmorfrequenz in einem sogenannten Stoppband des RF-Pulses. Da kein anwendbarer RF-Puls ein ideales Profil aufweist, existiert zwischen den Pass- und den Stoppbanden jeweils ein Übergangsbereich. Bei wiederholter Anwendung von RF-Pulsen jeweils nach einer Wiederholzeit TR, wie sie z.B. bei Steady-State-Pulssequenzen eingesetzt werden, kann sich darüber hinaus durch Off-Resonanzeffekte eine Bandstruktur in den Frequenzen der Signalantworten ausbilden, die weitere Stoppbanden innerhalb des eigentlichen Passbandes erzeugt, wodurch sogenannte Banding-Artefakte entstehen können. Die Bandstruktur, d.h. Anordnung der Frequenzbereiche, die Passbanden bilden und der Frequenzbereiche, die Stoppbanden bilden, ist hierbei nicht nur von den angewandten RF-Pulsen und der Ausgestaltung deren Einstrahlung, sondern auch von den Relaxationseigenschaften des untersuchten Untersuchungsobjektes, z.B. der Longitudinalrelaxation T1 und der Transversalrelaxation T2 der in dem Untersuchungsobjekt vorhandenen Gewebe, abhängig.

Zur Ortskodierung der Messdaten werden dem Grundmagnetfeld schnell geschaltete magnetische Gradientenfelder überlagert, die die Trajektorien festlegen, entlang derer die Messdaten im k-Raum ausgelesen werden. Die aufgezeichneten Messdaten werden digitalisiert und als komplexe Zahlenwerte in einer k-Raum-Matrix abgelegt. Aus der mit Werten belegten k-Raum-Matrix ist z.B. mittels einer mehrdimensionalen FourierTransformation ein zugehöriges MR-Bild rekonstruierbar. Eine hierzu verwendete auf bestimmte Art und Weise geordnete Folge von einzustrahlenden RF-Pulsen, zu schaltenden Gradienten und Auslesevorgängen wird als Pulssequenz bezeichnet.

Es sind verschiedene Pulssequenztypen bekannt, die unterschiedlich sensitiv auf die in einem untersuchten Untersuchungsobjekt enthaltenen Stoffe beschreibende Parameter (z.B. die Longitudinalrelaxation T1, die Transversalrelaxation T2 und die Protonendichte) sind. Die aus mit einem bestimmten Sequenztypen aufgenommenen Messdaten rekonstruierten MR-Bilder zeigen entsprechend der Sensitivitäten des verwendeten Sequenztyps gewichtete Abbildungen des Untersuchungsobjektes.

Eine Magnetresonanz-Bildgebung mittels einer Magnetresonanzanlage kann dazu dienen, eine Anwesenheit und/oder eine Verteilung eines Stoffs, welcher sich in einem Untersuchungsobjekt befindet, zu bestimmen. Der Stoff kann dabei beispielsweise ein, möglicherweise pathologisches, Gewebe des Untersuchungsobjekts, ein Kontrastmittel, eine Markierungssubstanz oder ein Stoffwechselprodukt sein.

Informationen über die vorliegenden Stoffe können dabei auf vielfältige Weise aus den aufgenommenen Messdaten gewonnen werden. Eine relativ einfache Informationsquelle sind z.B. aus den Messdaten rekonstruierte Bilddaten. Es gibt jedoch auch komplexere Verfahren, die, z.B. aus Bildpunkt-Zeit-Serien von aus sukzessive gemessenen Messdatensätzen rekonstruierten Bilddaten, Informationen über das untersuchte Untersuchungsobjekt ermitteln.

Mit Hilfe von quantitativen MR-Bildgebungstechniken lassen sich absolute Eigenschaften des gemessenen Objekts bestimmen, z.B. die gewebespezifische T1- und T2-Relaxation am Menschen. Im Gegensatz dazu erzeugen die in der klinischen Routine meist verwendeten konventionellen Sequenzen lediglich eine relative Signalintensität unterschiedlicher Gewebetypen (sogenannte Wichtungen), so dass die diagnostische Interpretation in hohem Maße der subjektiven Einschätzung des Radiologen unterliegt. Quantitative Techniken bieten somit den offensichtlichen Vorteil einer objektiven Vergleichbarkeit, finden aber aufgrund langer Messzeiten derzeit in der Routine kaum Verwendung.

Neuere quantitative Messverfahren wie Magnetresonanz-Fingerprinting-Verfahren (MRF-Verfahren), könnten den genannten Nachteil der langen Messzeiten auf ein akzeptables Maß senken. Bei MRF-Verfahren werden zeitlich nacheinander mit verschiedenen Aufnahmeparametern Messdaten aufgenommenen. Aus den nacheinander aufgenommenen Messdaten wird eine Serie von Bilddaten rekonstruiert. Ein Signalverlauf jeweils eines der Bildpunkte der Serien von Bilddaten wird als Bildpunkt-Zeit-Serie betrachtet. Hierbei kann der Signalverlauf für alle oder zumindest für interessierende Bildpunkte der Bilddaten untersucht werden. Ein solcher Signalverlauf einer Bildpunkt-Zeit-Serie wird hierbei oft als "Fingerabdruck" (engl. "fingerprint") des in dem jeweiligen Bildpunkt dargestellten Ortes des Untersuchungsobjektes bezeichnet. Ein derartiger Signalverlauf kann genutzt werden, um die während der Messung in dem durch den Bildpunkt dargestellten Ort des Untersuchungsobjekts vorliegenden Parameter zu bestimmen.

Dazu werden diese Signalverläufe mittels Mustererkennungsverfahren ("pattern recognition") mit Signalverläufen einer zuvor ermittelten Datenbank von für bestimmte Stoffe charakteristischen Signalverläufen (dem sogenannten "Dictionary") verglichen. Somit können die in den aus den Messdaten rekonstruierten Bilddaten repräsentierten Stoffe bzw. die räumliche Verteilung von gewebespezifischen Parametern (wie die Transversalrelaxation T2, die effektive Transversalrelaxation T2* oder die Longitudinalrelaxation T1; sogenannte T2, T2*- und T1-Karten) in dem abgebildeten Untersuchungsobjekt ermittelt werden. Die in einem derartigen Dictionary enthaltenen Signalverläufe können hierbei auch durch Simulationen erstellt worden sein.

Das Prinzip dieser Methode ist somit, gemessene Signalverläufe mit einer Vielzahl im Voraus bekannter Signalverläufe zu vergleichen. Dabei können Signalverläufe für verschiedene Kombinationen aus T1- und T2-Relaxationszeiten sowie auch anderen Parametern für das Dictionary ermittelt worden sein. Man spricht von je einer "Dimension" des Dictionarys für jeden zu bestimmenden Parameter, in der verschiedene Parameterwerte des jeweiligen Parameters umfasst sind, um verschiedene Vergleichswerte zur Verfügung zu stellen. Die Parameterwerte, z.B. T1- und T2-Zeiten, eines Bildpunktes (Pixels/Voxels) im Bild werden dann insbesondere bestimmt, indem der gemessene Signalverlauf mit allen oder einem Teil der simulierten Signalverläufe verglichen wird. Dieser Vorgang wird als "Matching" bezeichnet. Derjenige Signalverlauf des Dictionarys, der dem gemessenen Signalverlauf am ähnlichsten ist, determiniert in bekannten MRF-Verfahren die Parameter, z.B. Relaxationsparameter T1 und T2, des jeweiligen Bildpunktes. Eine derartige Bestimmung der Parameterwerte wird im Zusammenhang mit MRF-Techniken auch als Rekonstruktion bzw. Rekonstruktionsprozess bezeichnet.

Grundsätzlich können dabei neben den bereits genannten gewebespezifischen Parametern eines untersuchten Objektes auch messungsspezifische Parameter, wie z.B. die Feldstärken der applizierten Magnetfelder oder auch die lokale Verteilung der Stärke eines eingestrahlten Hochfrequenzfeldes B1, ermittelt werden, da mittels MR-Techniken aufgenommene Signale von den in einem untersuchten Objekt vorliegenden gewebespezifischen Parametern sowie von messungsspezifischen Parametern, die die während der Messung vorliegenden Bedingungen beschreiben, abhängen können. Die verwendeten Aufnahmeparameter sind hierbei derart gewählt, dass die aufgenommenen Messdaten eine Abhängigkeit von den gewünschten zu bestimmenden Parametern zeigen. Beispielsweise können Sequenztypen für das MRF-Verfahren eingesetzt werden, die sensitiv auf die gewünschten Parameter sind. Durch die Abhängigkeiten und die Variation der Aufnahmeparameter und deren Berücksichtigung in den Vergleichssignalverläufen sind die gewünschten Parameter aus derartig aufgenommenen Bildpunkt-Zeit-Serien bestimmbar.

Für MRF-Verfahren kann grundsätzlich jede Echotechnik (insbesondere Spinecho(SE)-Techniken und Gradientenecho(GRE)-Techniken) in Kombination mit jeglichen Verfahren zur k-Raumabtastung (z.B. kartesisch, spiralförmig, radial) benutzt werden.

Ein MRF-Verfahren, das die gewebespezifischen Parameter T1 und T2 in dem verwendeten Dictionary berücksichtigt und in gemessenen Bildpunkt-Zeit-Serien bestimmt, ist beispielsweise in dem Artikel von Ma et al., "Magnetic Resonance Fingerprinting", Nature, 495: S. 187-192 (2013) beschrieben. Dort wird eine TrueFISP ("true fast imaging with steady-state free precession") basierte Sequenz in Kombination mit einer spiralförmigen k-Raumabtastung benutzt.

Eine andere MRF-Implementierung wird von Jiang et al. in dem Artikel ""MR Fingerprinting Using Fast Imaging with Steady State Precession (FISP) with Spiral Readout", Magnetic Resonance in Medicine 74: S. 1621-1631, 2015, beschrieben. Dort wird eine FISP-Sequenz ("Fast Imaging with Steady State Precession") in Kombination mit einer Spiral-Abtastung verwendet: Nach einem adiabatischen 180° RF-Inversionspuls zur gezielten Störung des Gleichgewichtzustandes der Spins wird eine Folge von RF-Anregungspulsen mit pseudorandomisierten Flipwinkeln appliziert und jedes jeweils nach einem der RF-Anregungspulse resultierende Echo mit einer einzelnen spiralförmigen k-Raumtrajektorie ausgelesen. Es werden n RF-Anregungspulse benutzt, die ebenso viele Echos erzeugen. Aus den entlang der jeweiligen k-Raumtrajektorie aufgenommenen Messdaten jedes Echos wird ein Einzelbild rekonstruiert. Aus den n Einzelbildern wird für jeden Bildpunkt ein Signalverlauf extrahiert, der mit den simulierten Verläufen verglichen wird. Der zeitliche Abstand TR zwischen zwei aufeinanderfolgenden RF-Anregungspulsen der n RF-Anregungspulse kann hierbei ebenfalls, z.B. pseudorandomisiert, variiert werden.

Aufgrund der Vielzahl von veränderlichen Parametern ist es nicht einfach ein optimales Vorgehen für MRF-Verfahren zu bestimmen. Um MRF-Verfahren zu optimieren, sind jedoch bereits einige Versuche unternommen worden. So ist aus dem Artikel von Zhao Bo et al, "Optimal Experiment Design for Magnetic Resonance Fingerprinting", Conf. Proc. IEEE Eng. Med. Biol. Soc., 2016, beispielsweise bereits ein Ansatz bekannt, der versucht, die Kodierung der für einen Fingerprint aufzunehmenden Signale, und damit der erstellten Bildpunkt-Zeit-Serien, mit Bezug auf die bei MRF erforderliche Variation der Bildgebungsparameter und damit der nötigen Anzahl der aufzunehmenden Signale pro Fingerprint einerseits und der Fähigkeit der Fingerprints verschiedene Parameter zu quantifizieren andererseits, zu optimieren. Ein Verfahren zur Optimierung des bei MRF-Verfahren verwendeten Samplingschemas, also einer Verbesserung der Aufnahme der MR-Rohdaten bei MRF, ist beispielsweise in der Patentanmeldung US20180074145A1 beschrieben.

Der Erfindung liegt die Aufgabe zugrunde, eine effiziente quantitative Bestimmung von Parametern mittels MRF bei hoher Qualität der Ergebnisse zu ermöglichen.

Die Aufgabe wird gelöst durch ein Verfahren zur Bestimmung von Parameterwerten in Bildpunkten eines Untersuchungsobjektes mittels einer Magnetresonanz-Fingerprinting(MRF)-Technik gemäß Anspruch 1, eine Magnetresonanzanlage gemäß Anspruch 13, ein Computerprogramm gemäß Anspruch 14, sowie einen elektronisch lesbaren Datenträger gemäß Anspruch 15.

Ein erfindungsgemäßes Verfahren zur Bestimmung von Parameterwerten in Bildpunkten eines Untersuchungsobjektes mittels einer Magnetresonanz-Fingerprinting(MRF)-Technik umfasst die Schritte:
- Laden von zuvor erstellten Vergleichssignalverläufen (D),
- Erfassen mindestens einer Bildpunkt-Zeit-Serie (BZS) des Untersuchungsobjekts mit Hilfe eines MRF-Aufnahmeverfahrens an einer Niederfeld-Magnetresonanzanlage,
- Bestimmen der Werte (P) der zu bestimmenden Parameter auf Basis von Signalvergleichen mindestens eines Abschnitts des jeweiligen Signalverlaufs der erfassten Bildpunkt-Zeit-Serien (BZS) mit einem entsprechenden Abschnitt der geladenen Vergleichssignalverläufe (D),
- Speichern und/oder Ausgeben der für den jeweiligen Bildpunkt bestimmten Werte (P) der zu bestimmenden Parameter.

Bisher sind keine Verfahren bekannt, die MRF bei Niederfeld-Magnetresonanzanlagen einsetzten. Unter Niederfeld-Magnetresonanzanlagen werden hier Magnetresonanzanlagen verstanden, die Grundmagnetfeldstärken von kleiner als 1,5 Tesla, bevorzugt von kleiner als 0,5 Tesla, aufweisen.

Niederfeld-Magnetresonanzanlagen sind kostengünstig und benötigen, im Gegensatz zu Magnetresonanzanlagen mit höheren Grundmagnetfeldstärken (ab ca. 1,5 Tesla), nicht zwingend supra-leitende Magnete, wodurch sie grundsätzlich weniger Raum benötigen und leichter zu handhaben sind.

Erfindungsgemäß wird ein MRF-Verfahren zur Bestimmung von Parameterwerten in Bildpunkten eines Untersuchungsobjektes für Magnetresonanzanlagen mit einer Grundmagnetfeldstärke von kleiner als 1,5 Tesla, bevorzugt mit einer Grundmagnetfeldstärke von kleiner als 0,5 Tesla vorgeschlagen. Das MRF-Verfahren kann an die bei derartigen Niederfeld-Magnetresonanzanlagen herrschenden Bedingungen angepasst werden, sodass die Parameterwerte besonders effizient bei gleichzeitig hoher Qualität bestimmt werden können.

Niederfeld-Magnetresonanzanlagen können eine hohe Homogenität des tatsächlich erreichten Grundmagnetfeldes aufweisen. Des Weiteren ist eine über die spezifische Absorptionsrate (SAR, engl. "specific absorption rate") definierte Belastung eines Untersuchungsobjekts durch die kleinere Grundmagnetfeldstärke gegenüber Anlagen mit höheren Grundmagnetfeldstärken reduziert, sodass mehr Spielraum bei der Gestaltung der ebenfalls in den SAR-Wert eingehenden RF-Pulse genutzt, und Transmit-Felder B1 mit höheren Amplituden eingestrahlt werden können.

Bei der Erstellung der Vergleichssignalverläufe für ein erfindungsgemäßes MRF-Verfahren kann mindestens ein messungsspezifischer Parameter aus der Gruppe umfassend die Feldstärke eines während der Aufnahme der MR-Rohdaten anliegenden Grundmagnetfeldes B0 und die Feldstärke eines während der Aufnahme der MR-Rohdaten anliegenden Transmit-Feldes B1 nicht berücksichtigt worden sein. Ein auf diese Weise erstellter Dictionary aus Vergleichssignalverläufen ist durch den Verzicht auf die Dimension des Grundmagnetfeldes B0 und/oder auf die Dimension des Transmit-Feldes B1 deutlich kleiner als Dictionarys, die diese Dimensionen enthalten. Dadurch werden der für das durchzuführende Matching benötigte Rechenaufwand und die für das MRF-Verfahren benötigte Zeit erheblich reduziert.

Anstelle eines vollständigen Verzichts auf zumindest eine der Dimensionen Grundmagnetfeld B0 und Transmit-Feld B1 bei der Erstellung der Vergleichssignalverläufe des Dictionarys kann auch mindestens ein messungsspezifischer Parameter aus der Gruppe umfassend die Feldstärke eines während der Aufnahme der MR-Rohdaten anliegenden Grundmagnetfeldes B0 und die Feldstärke eines während der Aufnahme der MR-Rohdaten anliegenden Transmit-Feldes B1 in geringerem Maße berücksichtigt werden. Beispielsweise kann die Variation mindestens eines der messungsspezifischen Parameter, d.h. der Bereich aus welchem Werte für einen Vergleichssignalverlauf vorgesehen werden, klein gewählt werden. Es kann hierzu z.B. eine Variation von maximal 20 Prozent (z.B. ±10 Prozent), bevorzugt von maximal 5 Prozent (z.B. ±2,5 Prozent) um einen erwarteten relativen Wert (entspr. 100 Prozent) gewählt werden.

Die Variation eines der genannten Parameterwerte kann auch im Vergleich zu einer bei der Erstellung der Vergleichssignalverläufe verwendeten Variation von gewebespezifischen Parametern klein gewählt werden. Zusätzlich oder alternativ kann eine bei der Erstellung der Vergleichssignalverläufe verwendete Anzahl an Parameterwerten, für welche Vergleichssignalverläufe erstellt werden, von mindestens einem der messungsspezifischen Parameter deutlich kleiner gewählt werden, als eine Anzahl von Parameterwerten eines gewebespezifischen Parameters für die Vergleichssignalverläufe erstellt werden. Es kann bei der Erstellung der Vergleichssignalverläufe auch eine gewählte Auflösung der Parameterwerte in einem gewählten Parameterbereich, d.h. insbesondere die Anzahl der für die Erstellung der Vergleichssignalverläufe verwendeten Parameterwerte, für mindestens einen messungsspezifischen Parameter deutlich kleiner gewählt werden, als eine Auflösung von gewebespezifischen Parametern.

Bei der Erstellung der Vergleichssignalverläufe kann so mindestens einer der messungsspezifischen Parameter im Vergleich zu bei der Erstellung der Vergleichssignalverläufe berücksichtigten gewebespezifischen Parametern (= 100 Prozent Berücksichtigung) mit Bezug auf Anzahl der verwendeten Parameterwerte, Variation und/oder Auflösung nur zu maximal 20 Prozent, bevorzugt zu maximal 5 Prozent, berücksichtigt worden sein. Dadurch kann bereits eine erhebliche Reduzierung der Größe des Dictionarys erreicht werden, und so ebenfalls der Rechenaufwand und die Rechenzeit, die für das MRF-Verfahren benötigt werden, bereits reduziert werden.

Werden beispielsweise für gewebespezifische Parameter wie die Transversalrelaxation und die Longitudinalrelaxation etwa 50 bis 80 oder sogar 100 verschiedene Werte bei der Erstellung der Vergleichssignalverläufe berücksichtigt, können für die genannten messungsspezifischen Parameter bereits 10 oder weniger verschiedene Werte verwendet werden.

Obwohl die Ergebnisse von MRF-Verfahren stark davon abhängen, welche Parameter bei der Erstellung des Dictionarys wie genau berücksichtigt wurden, und somit zu welchen Parametern und in welchem Parameterbereich mit welcher Auflösung an möglichen Parameterwerten Vergleichssignalverläufe erstellt wurden, kann durch die Verwendung einer Niederfeld-Magnetresonanzanlage trotz der Verkleinerung des Dictionarys eine hohe Qualität der Bestimmung der Parameterwerte aufrechterhalten werden, indem z.B. über das Ausmaß einer derartigen Verkleinerung des Dictionarys gemäß der durch die Niederfeld-Magnetresonanzanlage verwirklichten Homogenität des Grundmagnetfeldes B0 und der durch die Niederfeld-Magnetresonanzanlage verwirklichten Homogenität des Transmit-Feldes B1 entschieden wird. Auf diese Weise kann die Erstellung der Vergleichssignalverläufe des Dictionarys für das durchzuführende MRF-Verfahren an die für die verwendete Niederfeld-Magnetresonanzanlage geltenden Bedingungen, insbesondere deren Hardware-Bedingungen, angepasst werden, sodass die für Niederfeld-Magnetresonanzanlagen geltenden spezifischen Bedingungen optimal genutzt werden können.

Die zur Aufnahme der MR-Rohdaten verwendete Pulssequenz kann eine Pulssequenz vom Typ einer ausbalancierten Steady-State-Freie-Präzession(bSSFP; auch TrueFISP genannt)-Pulssequenz sein. Insbesondere können die MR-Rohdaten überwiegend oder sogar ausschließlich mittels Pulssequenzen von Typ einer bSSFP-Pulssequenz aufgenommen werden. bSSFP-Pulssequenzen erlauben eine schnelle Aufnahme von MR-Rohdaten mit einem intrinsisch hohen Signal-Rausch-Verhältnis (SNR, engl. "signal-to-noise ratio"). Des Weiteren sind bSSFP-Pulssequenzen sensitiv sowohl auf die longitudinale als auch auf die transversale Relaxation, sodass mittels bSSFP-Pulssequenzen erhaltene Bildpunkt-Zeit-Serien gut unterscheidbar sind, d.h. dass diese beiden Parameter bereits aus mittels bSSFP-Pulssequenzen erhaltenen Bildpunkt-Zeit-Serien durch Vergleichen mit einem entsprechenden Dictionary mit hoher Genauigkeit bestimmbar sind. Durch die erfindungsgemäße Aufnahme der MR-Rohdaten mit einer Niederfeld-Magnetresonanzanlage werden darüber hinaus sonst häufig bei bSSFP-Pulssequenzen auftretende z.B. durch Inhomogenitäten des Grundmagnetfeldes erzeugte, sogenannte "Banding-Artefakte" vermieden. Durch das intrinsisch hohe SNR der bSSFP-Pulssequenzen kann trotz einer kleinen Grundmagnetfeldstärke eine gute Signalqualität erreicht werden.

Die Aufnahme der MR-Rohdaten kann hierbei entlang von kartesischen, radialen oder spiralen k-Raumtrajektorien erfolgen. In Verbindung mit einem kartesischen oder radialen Abtastschema sind sehr kurze Wiederholzeiten TRᵢ möglich, was die Messzeit kurz hält. Auch in Verbindung mit einer spiralförmigen Abtastung des k-Raums sind kurze Wiederholzeiten TRᵢ denkbar. Durch eine kurze Auslesedauer von zum Aufnehmen von als MR-Rohdaten zu speichernden Signalen verwendeten spiralen k-Raumtrajektorien und der bei Niederfeld-Magnetresonanzanlagen hohen Homogenität des Grundmagnetfeldes B0 ist der ansonsten zu erwartende Effekt von Spiralblurring nur gering, sodass wenig oder keine Spiralblurring-Artefakte auftreten.

Die zur Aufnahme der MR-Rohdaten verwendeten Flipwinkel, Echozeiten und/oder Wiederholungszeiten derart optimiert wurden, dass die angeregten Spins in einem Pseudo Steady-State vorlagen. Eine derartige Optimierung ist in dem Artikel von Assländer et al., "Pseudo Steady-State Free Precession for MR-Fingerprinting", Magnetic Resonance in Medicine 77: S. 1151-1161, 2017, beschrieben. Durch die derartige Erzeugung eines Pseudo Steady-States der Magnetisierung wird die Spin-Echo-Artigkeit der erzeugten Echosignale für MRF wiederhergestellt.

Im Rahmen des erfindungsgemäßen Verfahrens erfasste Bildpunkt-Zeit-Serien können aus MR-Rohdaten erstellt werden, die mittels einer einzustrahlende RF-Pulse und zu schaltende Gradienten umfassenden Pulssequenz aufgenommen wurden, bei der die angewandten Wiederholzeiten so gewählt sind, dass Stoppbanden innerhalb eines durch die Pulssequenz angeregten Anregungsbereichs in dem Untersuchungsobjekt vermieden werden. Auf diese Weise wird eine hohe Robustheit gegen Fluss und Bewegung innerhalb des Zielvolumens, z.B. einer Schicht, erreicht. Eine Aufnahme der MR-Rohdaten für das MRF-Verfahren bei Niederfeld-Magnetresonanzanlagen mit derart gewählten Wiederholzeiten TRᵢ dass Stoppbanden und damit durch Off-Resonanzeffekte erzeugte Artefakte vermieden werden, liefert eine hohe Qualität der aufgenommenen MR-Rohdaten. Stoppbanden werden z.B. reduziert, indem die Wiederholzeit TR verkleinert wird, wodurch Passbanden und auch mögliche Nebenbandenverbreitert werden.

Beispielsweise können hierzu die Wiederholzeiten TRᵢ so gewählt werden, dass das zentrale Passband der verwendeten, z.B. TrueFISP basierten Pulssequenz breit genug wird, um möglichst alle Spins im abzubildenden Untersuchungsbereich im zentralen Passband zu halten. Dazu können die Wiederholzeiten TRᵢ insbesondere so klein gewählt werden, dass das zentrale Passband der Pulssequenz, dessen Breite proportional zu 1/TRᵢ ist, die hierzu notwendige Breite erreicht.

Die Wiederholzeit TRᵢ kann für die Aufnahme der MR-Rohdaten insbesondere derart gewählt werden, dass ein Passband, insbesondere das zentrale Passband, der bei der Aufnahme der MR-Rohdaten verwendeten RF-Pulse breit genug ist, dass sowohl Spins einer ersten in dem Untersuchungsobjekt vorhandenen Spinspezies als auch Spins einer zweiten in dem Untersuchungsobjekt vorhandenen Spinspezies im Passband liegen, wobei die erste Spinspezies eine andere Resonanzfrequenz hat als die zweite Spinspezies. Auf diese Weise kann vermieden werden, dass das Signal einer Spinspezies zu niedrig ist. Über die Wiederholzeit ist nur die Breite der Passbänder, nicht aber die Lage der Passbänder im Frequenzbereich beeinflussbar.

Zur Einstellung der Lage der Passbänder kann der Phasenzyklus von bei Aufnahme der MR-Rohdaten verwendeten RF-Pulsen derart gewählt werden, dass sowohl Spins einer ersten in dem Untersuchungsobjekt vorhandenen Spinspezies als auch Spins einer zweiten in dem Untersuchungsobjekt vorhandenen Spinspezies in einem Passband, insbesondere dem zentralen Passband, angeregt werden, wobei die erste Spinspezies eine andere Resonanzfrequenz hat als die zweite Spinspezies.

Die erste und die zweite Spinspezies können beispielsweise aus der Gruppe der Spinspezies Fett und Wasser gewählt sein, deren Resonanzfrequenzen unterschiedlich sind. Der Unterschied der Resonanzfrequenzen von Fett und Wasser wird i.A. als "chemical shift" bezeichnet. Dieser hängt linear von der Feldstärke ab, wobei der Unterschied bei niedrigen Frequenzen kleiner ist. Bei Niederfeld-MR ergibt sich also der Vorteil, dass leichter kurze TR gefunden werden können, bei denen Fett und Wasser im zentralen Passband liegen.

Eine erfindungsgemäße Magnetresonanzanlage umfasst eine Magneteinheit, eine Gradienteneinheit, eine Hochfrequenzeinheit und eine zur Durchführung eines erfindungsgemäßen Verfahrens ausgebildete Steuereinrichtung mit einer Parameterwertbestimmungseinheit.

Ein erfindungsgemäßes Computerprogramm implementiert ein erfindungsgemäßes Verfahren auf einer Steuereinrichtung, wenn es auf der Steuereinrichtung ausgeführt wird.

Das Computerprogramm kann hierbei auch in Form eines Computerprogrammprodukts vorliegen, welches direkt in einen Speicher einer Steuereinrichtung ladbar ist, mit Programmcode-Mitteln, um ein erfindungsgemäßes Verfahren auszuführen, wenn das Computerprogrammprodukt in der Recheneinheit des Rechensystems ausgeführt wird.

Ein erfindungsgemäßer elektronisch lesbarer Datenträger umfasst darauf gespeicherte elektronisch lesbare Steuerinformationen, welche zumindest ein erfindungsgemäßes Computerprogramm umfassen und derart ausgestaltet sind, dass sie bei Verwendung des Datenträgers in einer Steuereinrichtung einer Magnetresonanzanlage ein erfindungsgemäßes Verfahren durchführen.

Die in Bezug auf das Verfahren angegebenen Vorteile und Ausführungen gelten analog auch für die Magnetresonanzanlage, das Computerprogramm und den elektronisch lesbaren Datenträger.

Weitere Vorteile und Einzelheiten der vorliegenden Erfindung ergeben sich aus den im Folgenden beschriebenen Ausführungsbeispielen sowie anhand der Zeichnungen. Die aufgeführten Beispiele stellen keine Beschränkung der Erfindung dar. Es zeigen:
- Fig. 1: ein schematisches Ablaufdiagramm eines erfindungsgemäßen Verfahrens,
- Fig. 2: ein grob schematisch dargestelltes Pulssequenzdiagramm, zur Veranschaulichung von Pulssequenzen wie sie für ein erfindungsgemäßes Verfahren eingesetzt werden können,
- Fig. 3: eine schematisch dargestellte erfindungsgemäße Magnetresonanzanlage.

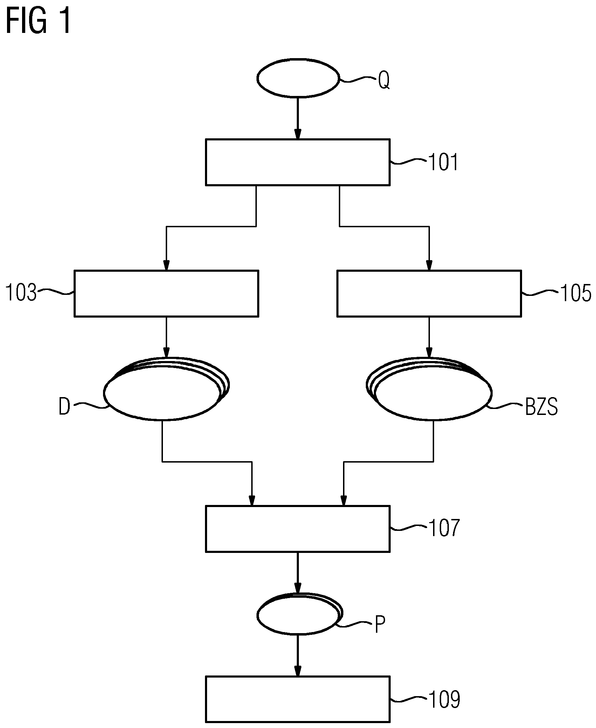

Figur 1 ist ein schematisches Ablaufdiagramm eines erfindungsgemäßen Verfahrens zur Bestimmung von Parameterwerten in Bildpunkten eines Untersuchungsobjektes mittels einer Magnetresonanz-Fingerprinting(MRF)-Technik.

Dabei werden zuvor erstellte Vergleichssignalverläufe D geladen (Block 103).

Mit Hilfe eines MRF-Aufnahmeverfahrens an einer Niederfeld-Magnetresonanzanlage mit einem Grundmagnetfeld von kleiner als 1,5 Tesla, bevorzugt mit einem Grundmagnetfeld von kleiner als 0,5 Tesla, wird mindestens eine Bildpunkt-Zeit-Serie BZS erfasst (Block 105).

Wie bei MRF-Verfahren üblich, werden Werte P der zu bestimmenden Parameter, wie beispielsweise die dem Bildpunkt entsprechenden lokalen Werte der Longitudinalrelaxation oder der Transversalrelaxation, auf Basis von Signalvergleichen (insbesondere im Rahmen eines MRF-Matching-Verfahrens) mindestens eines Abschnitts des jeweiligen Signalverlaufs einer erfassten Bildpunkt-Zeit-Serie BZS mit einem entsprechenden Abschnitt der geladenen Vergleichssignalverläufe D bestimmt (Block 107).

Die bestimmten Werte P der zu bestimmenden Parameter können gespeichert und/oder weiterverarbeitet, z.B. in Form einer Parameterkarte einer Anzeigevorrichtung wie einem Display ausgegeben werden.

Sowohl bei der Erstellung der Vergleichssignalverläufe D in Block 103, als auch bei der Erfassung der Bildpunkt-Zeit-Serien BZS in Block 105 können bei der durchzuführenden MRF-Messung geltende Bedingungen Q berücksichtigt werden (Block 101). Derartige Bedingungen können insbesondere Hardwareeigenschaften der Magnetresonanzanlage, auf der die MRF-Messung durchgeführt werden soll, sein, wie z.B. Eigenschaften aus der Gruppe umfassend Grundmagnetfeldstärke und Gradienteneigenschaften wie mögliche Rampengeschwindigkeit und mögliche Gradientenstärke. Auf Grundlage dieser Bedingungen Q können, z.B. wie bereits oben beschrieben, die Dimensionen (welche Parameter variiert werden) und die Größe der jeweiligen Dimensionen (in welchem Bereich und mit welcher Auflösung die jeweiligen Parameter variiert werden) festgelegt werden. Des Weiteren können die bei dem MRF-Verfahren zu verwendenden Pulssequenzparameter, wie Flipwinkel, Wiederholzeiten, Echozeiten, auf Grundlage der Bedingungen Q optimiert gewählt werden.

Hierbei kann, insbesondere wie bereits weiter oben beschrieben, mindestens ein eine Dimension des Dictionarys bildender messspezifischer Parameter bei der Erstellung der Vergleichssignalverläufe des Dictionarys nicht oder nur in geringem Maße berücksichtigt werden.

Somit kann bei der Erstellung der Vergleichssignalverläufe mindestens ein messungsspezifischer Parameter aus der Gruppe umfassend die Feldstärke eines während der Aufnahme der MR-Rohdaten anliegenden Grundmagnetfeldes B0 und die Feldstärke eines während der Aufnahme der MR-Rohdaten anliegenden Transmit-Feldes B1 nicht berücksichtigt werden.

Ebenso kann bei der Erstellung der Vergleichssignalverläufe mindestens ein messungsspezifischer Parameter aus der Gruppe umfassend die Feldstärke eines während der Aufnahme der MR-Rohdaten anliegenden Grundmagnetfeldes B0 und die Feldstärke eines während der Aufnahme der MR-Rohdaten anliegenden Transmit-Feldes B1 im Vergleich zu gewebespezifischen Parametern deutlich geringer berücksichtigt werden.

Nicht nur das verwendete Dictionary mit seinen Vergleichssignalverläufen kann für Niederfeld-Magnetresonanzanlagen besonders effizient gestaltet werden. Auch die Kodierung der als MR-Rohdaten aufzunehmenden Signale kann für Niederfeld-Magnetresonanzanlagen derart gestaltet werden, dass eine hohe Signalqualität, insbesondere eine besonders geringe Anfälligkeit für Artefakte, erreicht wird.

Beispielsweise können erfasste Bildpunkt-Zeit-Serien BZS aus MR-Rohdaten erstellt werden, die mittels einer einzustrahlende RF-Pulse und zu schaltende Gradienten umfassenden Pulssequenz aufgenommen wurden, bei der wie bereits weiter oben beschrieben die angewandten Wiederholzeiten TRᵢ so gewählt sind, dass Stoppbanden innerhalb eines durch die Pulssequenz angeregten Anregungsbereichs in dem Untersuchungsobjekt vermieden werden.

Als die zur Aufnahme der MR-Rohdaten verwendete Pulssequenz kann eine Pulssequenz vom Typ einer bSSFP-Pulssequenz, auch eine TrueFISP-artige Pulssequenz genannt, verwendet werden. Neben den bereits weiter oben beschriebenen Vorteilen, erlaubt diese auch sehr kurze Wiederholzeiten, z.B. Wiederholzeiten von deutlich unter 10 Millisekunden, beispielsweise Wiederholzeiten von nur 2 Millisekunden, sodass die Passbanden der RF-Pulse besonders breit gestaltet werden können.

Die MR-Rohdaten, aus denen die Bildpunkt-Zeit-Serien erstellt werden, können entlang von kartesischen, radialen oder spiralen k-Raumtrajektorien aufgenommen werden. Die Wahl der zu verwendenden k-Raumtrajektorien kann z.B. von den jeweiligen Abtastschemata eigenen Artefaktanfälligkeiten und der gewünschten Signalqualität abhängig gemacht werden.

Wie bereits oben beschrieben, können die zur Aufnahme der MR-Rohdaten verwendeten Flipwinkel, Echo- und/oder Wiederholungszeiten derart optimiert gewählt werden, dass die angeregten Spins während der Aufnahme der MR-Rohdaten in einem Pseudo Steady-State vorliegen. Damit kann z.B. die Unterscheidbarkeit verschiedener Werte für die Longitudinalrelaxation verbessert werden. Das hierzu in dem bereits genannten Artikel von Assländer et al. beschriebene Verfahren funktioniert in dem zentralen Passband der verwendeten Pulssequenz, dessen Breite über eine Wahl der Wiederholzeiten TRᵢ wie bereits beschrieben besonders breit gestaltet werden kann.

Über die Wahl der verwendeten Wiederholzeiten TRᵢ, z.B. durch eine Einschränkung auf kleine Werte, können die Passbanden der verwendeten RF-Pulse derart breit gestaltet werden, dass ein Passband, insbesondere das zentrale Passband, der bei der Aufnahme der MR-Rohdaten verwendeten RF-Pulse breit genug ist, dass sowohl Spins einer ersten in dem Untersuchungsobjekt vorhandenen Spinspezies als auch Spins einer zweiten in dem Untersuchungsobjekt vorhandenen Spinspezies in einem Passband, insbesondere dem zentralen Passband, angeregt werden können, wobei die erste Spinspezies eine andere Resonanzfrequenz hat als die zweite Spinspezies. Somit werden destruktive Signalüberlagerungen und damit Artefakte vermieden, wobei gleichzeitig Signale der beiden Spinspezies optimal in den MR-Rohdaten enthalten sind. Die Breite des Passbandes ist kleiner als der Kehrwehrt der verwendeten Wiederholzeit TR ("maximale gewünschte Resonanzfrequenz" - "minimale gewünschte Resonanzfrequenz" < 1/TR).

Dabei kann zusätzlich oder alternativ der Phasenzyklus und/oder die Frequenz, insbesondere die Mittenfrequenz und/oder die Bandbreite, der bei Aufnahme der MR-Rohdaten verwendeten RF-Pulse derart gewählt werden, dass sowohl Spins einer ersten in dem Untersuchungsobjekt vorhandenen Spinspezies als auch Spins einer zweiten in dem Untersuchungsobjekt vorhandenen Spinspezies in einem Passband, insbesondere dem zentralen Passband, angeregt werden, wobei die erste Spinspezies eine andere Resonanzfrequenz hat als die zweite Spinspezies. Eine derartige Wahl von Phasenzyklus und/oder Frequenz der verwendeten RF-Pulse kann bereits ausreichend sein, um die Resonanzfrequenzen beider gewünschter Spinspezies im Passband abzudecken. In jedem Fall kann über eine derartige Wahl erreicht werden, dass die Breite des Passbandes nicht unnötig breit gestaltet werden muss, sondern den Bereich der gewünschten Resonanzfrequenzen (maximale Resonanzfrequenz bis minimale Resonanzfrequenz im Untersuchungsobjekt) gerade abgedeckt wird.

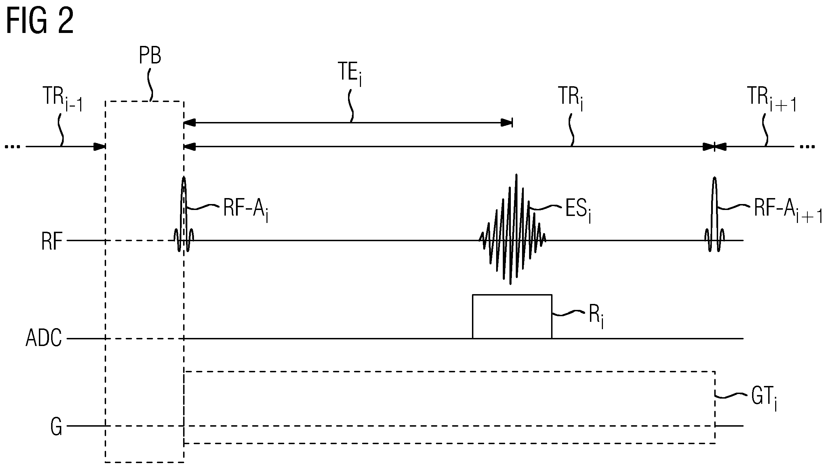

Ist es nicht gewünscht oder möglich die Signale von zwei verschiedenen Spinspezies aufzunehmen, können die Signale einer der beiden Spinspezies auch unterdrückt werden. Hierzu wird weiter unten mit Bezug auf Figur 2 Weiteres ausgeführt.

Ein grob schematisches Beispiel anwendbarer Pulssequenzen ist in Figur 2 anhand eines Pulssequenzdiagramms dargestellt. Als Pulssequenz wird hier allgemein dasjenige Schema einer zeitlichen Abfolge von einzustrahlenden RF-Pulsen und zu schaltenden Gradienten bezeichnet, die zur Erzeugung und zum Auslesen von MR-Signalen eines Untersuchungsobjektes verwendet werden. Gemäß der Pulssequenz werden somit RF-Pulse in ein Untersuchungsobjekt eingestrahlt, Gradienten geschaltet und durch die eingestrahlten RF-Pulse und die geschalteten Gradienten erzeugte Echosignalen ausgelesen.

In der obersten Zeile (RF) ist die Hochfrequenzaktivität dargestellt. Innerhalb einer Wiederholzeit TRᵢ wird zumindest ein Anregungspuls RF-Aᵢ eingestrahlt und nach einer Echozeit TEᵢ nach dem Anregungspuls RF-Aᵢ ein Echosignal ESᵢ in einem Auslesezeitraum Rᵢ als MR-Rohdaten ausgelesen (ADC). Je nach Pulssequenz können zwischen dem Anregungspuls RF-Aᵢ und dem Echosignal ESᵢ auch noch weitere RF-Pulse geschaltet werden, auf deren Darstellung der Übersichtlichkeit halber verzichtet wurde. Innerhalb einer Wiederholzeit TRᵢ wird ein Gradientenzug GTᵢ, der Gradienten in allen Gradientenrichtungen G umfassen kann, geschaltet, dessen genaue Ausgestaltung wiederum von der Art der für diese Wiederholung gewählten Pulssequenz, z.B. einer Gradientenechosequenz, einer Spinechosequenz oder einer Steady-State-Sequenz, sowie von der k-Raum-Trajektorie abhängt, entlang derer die MR-Rohdaten ausgelesen werden sollen. Die für die verschiedenen bekannten Pulssequenztypen zu schaltenden Gradienten sind dem Fachmann grundsätzlich bekannt, beispielsweise wäre der Gradientenzug GTᵢ im Falle einer Pulssequenz von Typ einer balancierten Steady-State-Freie-Präzession (bSSFP, engl. "steady state free precession") während der Wiederholzeit TRᵢ derart ausgestaltet, dass das nullte Moment aller der bSSFP-Sequenz zugehörigen Gradienten kompensiert ist. Mit anderen Worten verschwindet das nullte Moment zwischen zwei aufeinanderfolgenden RF-Anregungspulsen RF-Aᵢ und RF-Aᵢ₊ᵢ. In Figur 2 ist der Gradientenzug GTᵢ zur Vereinfachung der Darstellung nur durch einen Kasten angedeutet. Nach einer Wiederholzeit TRᵢ, die erfindungsgemäß, z.B. wie in dem oben genannten Artikel von Asslaender et al. , variieren kann, wird erneut eine Pulssequenz gestartet, um weitere MR-Rohdaten aufzunehmen, wie durch den folgenden Anregungspuls RF-Aᵢ₊₁ angedeutet ist, wobei in jeder Wiederholung i andere Gradienten in dem Gradientenzug GTᵢ geschaltet und/oder andere RF-Pulse eingestrahlt werden können, und wobei auch in verschiedenen Wiederholungen i verschiedene Pulssequenztypen eingesetzt werden können.

Bei MRF-Verfahren kann in jeder Wiederholzeit TRᵢ ein Flipwinkel eingestrahlter RF-Pulse, insbesondere des Anregungspulses RF-Aᵢ variiert werden. Insgesamt werden bei MRF-Verfahren N Wiederholungen i durchgeführt (i=1, ..., N), wobei N eine natürliche Zahl größer als Eins ist und, je nach Anwendung, auch mehrere hundert bis mehrere tausend sein kann, sodass N-mal MR-Rohdaten aufgenommen werden. Aus jedem der N aufgenommenen Sätze an MR-Rohdaten kann zumindest für einen Bildpunkt ein Signalwert berechnet werden, wodurch man eine Bildpunkt-Zeit-Serie von zu N Zeiten aufgenommenen Signalwerten erhält.

Eine Folge an N derartigen Wiederholungen i kann gegebenenfalls durch Einfügen von Präparationsblöcken unterbrochen werden. Dies ist in Figur 2 durch einen Präparationsblock PB dargestellt, der zwischen der (i-1) -ten und der i-ten Wiederholung eingefügt ist. Während eines Präparationsblocks PB wird die Magnetisierung der untersuchten Spins in einer gewünschten Weise präpariert. Dazu können weitere RF-Pulse eingestrahlt und auch weitere Gradienten geschaltet werden. Beispielsweise kann ein RF-Inversionspuls eingestrahlt werden, um eine auch unter dem Überbegriff "Inversion Recovery" bekannte Präparation der Spins vorzunehmen, und beispielsweise Signale einer ausgewählten Spinspezies (z.B. Fett oder Flüssigkeit) zu unterdrücken. Es ist auch möglich Präparationsblöcke PB zur Präparation einer gewünschten Gewichtung, z.B. einer T1- oder einer T2-Präparation, zu verwenden. Die Aufnahme der MR-Rohdaten kann somit Präparationsblöcke umfassen.

Derartige Präparationsblöcke PB können insbesondere derart zwischen Wiederholungen (i-1) und i geschaltet werden, dass der Ablauf der N Wiederholungen periodisch von einem Präparationsblock PB unterbrochen wird. Die Präparationsblöcke können somit während der Aufnahme der MR-Rohdaten periodisch in der Zeit angeordnet sein. Hierdurch kann eine Wirkung der Präparationsblöcke, z.B. jeweils über mehrere auf einen Präparationsblock PB folgende Wiederholungen i, aufrechterhalten werden, wobei es nicht notwendig ist, vor jeder Wiederholung i einen Präparationsblock PB zu schalten. So kann die Messdauer insgesamt kleiner gehalten werden und z.B. erreichte Steady-States werden weniger unterbrochen.

Beispielsweise in dem Artikel von Koktzoglou et al., "Radial Fast Interrupted Steady-State (FISS) Magnetic Resonance Imaging", Magnetic Resonance in Medicine 79: S. 2077-2086, 2018, wird ein Verfahren beschrieben, dass durch Zwischenschalten von Unterbrechungen, die Rückstände einer transversale Magnetisierung im Untersuchungsvolumen zerstört, in Pulssequenzen vom Typ einer bSSFP-Sequenz (Fluss-)Artefakte vermeidet und eine Sättigung von Fettsignalen erreichen kann.

Es kann jedoch auch sinnvoll sein, Präparationsblöcke PB nicht-periodisch anzuordnen.

Sollen bei einer Aufnahme von MR-Rohdaten Signale einer indem Untersuchungsobjekt vorhandenen Spinspezies, beispielsweise der Spinspezies Fett, unterdrückt werden, können zusätzlich oder alternativ zu den bereits beschriebenen Präparationsblöcken PB; Binomialpulse, z.B. als RF-Anregungspulse, verwendet werden, die derart ausgestaltet sind, dass sie spektral selektiv nicht auf die zu unterdrückende Spinspezies wirken. Derartige Binomialpulse sind grundsätzlich bekannt. Bei der Verwendung von Binomialpulsen zur Unterdrückung von Signalen einer Spinspezies wird die Messzeit insgesamt nicht so wie bei einer Einfügung von Präparationsblöcken PB verlängert.

Die Qualität der erreichten Unterdrückung kann durch Kombination verschiedener Unterdrückungstechniken, z.B. Verwendung von Binomialpulsen und Präparationsblöcken PB, erhöht werden und/oder die für eine ausreichende Unterdrückung benötigte Häufigkeit von Präparationsblöcken PB durch Binomialpulse reduziert werden.

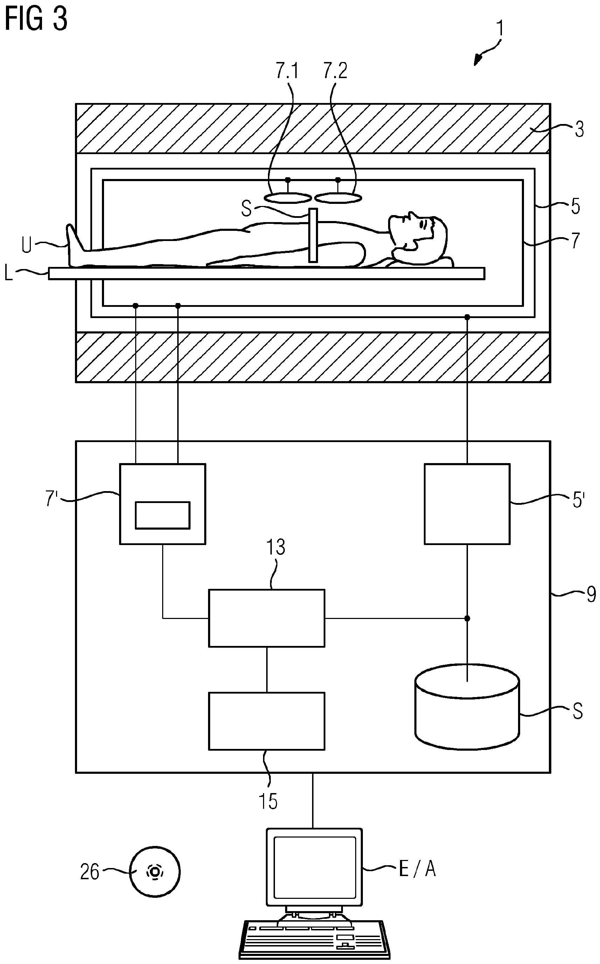

Figur 3 stellt schematisch eine erfindungsgemäße Magnetresonanzanlage 1 dar. Diese umfasst eine Magneteinheit 3 zur Erzeugung des Grundmagnetfeldes, eine Gradienteneinheit 5 zur Erzeugung der Gradientenfelder, eine Hochfrequenzeinheit 7 zur Einstrahlung und zum Empfang von Hochfrequenzsignalen und eine zur Durchführung eines erfindungsgemäßen Verfahrens ausgebildete Steuereinrichtung 9.

In der Figur 3 sind diese Teileinheiten der Magnetresonanzanlage 1 nur grob schematisch dargestellt. Insbesondere kann die Hochfrequenzeinheit 7 aus mehreren Untereinheiten, beispielsweise aus mehreren Spulen wie den schematisch gezeigten Spulen 7.1 und 7.2 oder mehr Spulen bestehen, die entweder nur zum Senden von Hochfrequenzsignalen oder nur zum Empfangen der ausgelösten Hochfrequenzsignale oder für beides ausgestaltet sein können.

Zur Untersuchung eines Untersuchungsobjektes U, beispielsweise eines Patienten oder auch eines Phantoms, kann dieses auf einer Liege L in die Magnetresonanzanlage 1 in deren Messvolumen eingebracht werden. Die Schicht S stellt ein exemplarisches Zielvolumen des Untersuchungsobjekts dar, aus dem Messdaten aufgenommen werden sollen.

Die Steuereinrichtung 9 dient der Steuerung der Magnetresonanzanlage und kann insbesondere die Gradienteneinheit 5 mittels einer Gradientensteuerung 5' und die Hochfrequenzeinheit 7 mittels einer Hochfrequenz-Sende-/Empfangs-Steuerung 7' steuern. Die Hochfrequenzeinheit 7 kann hierbei mehrere Kanäle umfassen, auf denen Signale gesendet oder empfangen werden können.

Die Hochfrequenzeinheit 7 ist zusammen mit ihrer Hochfrequenz-Sende-/Empfangs-Steuerung 7' für die Erzeugung und das Einstrahlen (Senden) eines Hochfrequenz-Wechselfeldes zur Manipulation der Spins in einem zu manipulierenden Bereich (beispielsweise in zu messenden Schichten S) des Untersuchungsobjekts U zuständig. Dabei wird die Mittenfrequenz des, auch als B1-Feld bezeichneten, Hochfrequenz-Wechselfeldes in aller Regel möglichst so eingestellt, dass sie nahe der Resonanzfrequenz (Larmorfrequenz) der zu manipulierenden Spins liegt. Zur Erzeugung des B1-Feldes (Transmit-Feldes) werden in der Hochfrequenzeinheit 7 mittels der Hochfrequenz-Sende-/Empfangs-Steuerung 7' gesteuerte Ströme an den HF-Spulen angelegt.

Weiterhin umfasst die Steuereinrichtung 9 eine Parameterwertbestimmungseinheit 15, mit welcher erfindungsgemäße Signalvergleiche zur Bestimmung von Parameterwerten durchgeführt werden können. Die Steuereinrichtung 9 ist insgesamt dazu ausgebildet, ein erfindungsgemäßes Verfahren durchzuführen.

Eine von der Steuereinrichtung 9 umfasste Recheneinheit 13 ist dazu ausgebildet alle für die nötigen Messungen und Bestimmungen nötigen Rechenoperationen auszuführen. Hierzu benötigte oder hierbei ermittelte Zwischenergebnisse und Ergebnisse können in einer Speichereinheit S der Steuereinrichtung 9 gespeichert werden. Die dargestellten Einheiten sind hierbei nicht unbedingt als physikalisch getrennte Einheiten zu verstehen, sondern stellen lediglich eine Untergliederung in Sinneinheiten dar, die aber auch z.B. in weniger oder auch in nur einer einzigen physikalischen Einheit realisiert sein können.

Über eine Ein-/Ausgabeeinrichtung E/A der Magnetresonanzanlage 1 können, z.B. durch einen Nutzer, Steuerbefehle an die Magnetresonanzanlage geleitet werden und/oder Ergebnisse der Steuereinrichtung 9 wie z.B. Bilddaten angezeigt werden.

Ein hierin beschriebenes Verfahren kann auch in Form eines Computerprogrammprodukts vorliegen, welches ein Programm umfasst und das beschriebene Verfahren auf einer Steuereinrichtung 9 implementiert, wenn es auf der Steuereinrichtung 9 ausgeführt wird. Ebenso kann ein elektronisch lesbarer Datenträger 26 mit darauf gespeicherten elektronisch lesbaren Steuerinformationen vorliegen, welche zumindest ein solches eben beschriebenes Computerprogrammprodukt umfassen und derart ausgestaltet sind, dass sie bei Verwendung des Datenträgers 26 in einer Steuereinrichtung 9 einer Magnetresonanzanlage 1 das beschriebene Verfahren durchführen.

## Patentansprüche

1. Verfahren zur Bestimmung von Parameterwerten in Bildpunkten eines Untersuchungsobjektes mittels einer Magnetresonanz-Fingerprinting(MRF)-Technik, umfassend die Schritte:
- Laden von zuvor erstellten Vergleichssignalverläufen (D),
- Erfassen mindestens einer Bildpunkt-Zeit-Serie (BZS) des Untersuchungsobjekts mit Hilfe eines MRF-Aufnahmeverfahrens an einer Niederfeld-Magnetresonanzanlage,
- Bestimmen der Werte (P) der zu bestimmenden Parameter auf Basis von Signalvergleichen mindestens eines Abschnitts des jeweiligen Signalverlaufs der erfassten Bildpunkt-Zeit-Serien (BZS) mit einem entsprechenden Abschnitt der geladenen Vergleichssignalverläufe (D),
- Speichern und/oder Weiterverarbeiten der für den jeweiligen Bildpunkt bestimmten Werte (P) der zu bestimmenden Parameter.

2. Verfahren nach Anspruch 1, wobei bei der Erstellung der Vergleichssignalverläufe mindestens ein messungsspezifischer Parameter aus der Gruppe umfassend die Feldstärke eines während der Aufnahme der MR-Rohdaten anliegenden Grundmagnetfeldes B0 und die Feldstärke eines während der Aufnahme der MR-Rohdaten anliegenden Transmit-Feldes B1 nicht berücksichtigt wurde.

3. Verfahren nach einem der vorhergehenden Ansprüche, wobei bei der Erstellung der Vergleichssignalverläufe mindestens ein messungsspezifischer Parameter aus der Gruppe umfassend die Feldstärke eines während der Aufnahme der MR-Rohdaten anliegenden Grundmagnetfeldes B0 und die Feldstärke eines während der Aufnahme der MR-Rohdaten anliegenden Transmit-Feldes nur in geringem Maße berücksichtigt wurde.

4. Verfahren nach einem der vorhergehenden Ansprüche, wobei die zur Aufnahme der MR-Rohdaten verwendete Pulssequenz eine Pulssequenz vom Typ einer ausbalancierten Steady-State-Freie-Präzession(bSSFP; TrueFISP)-Pulssequenz ist.

5. Verfahren nach einem der vorhergehenden Ansprüche, wobei die MR-Rohdaten entlang von kartesischen, radialen oder spiralen k-Raumtrajektorien aufgenommen wurden.

6. Verfahren nach einem der vorhergehenden Ansprüche, wobei die zur Aufnahme der MR-Rohdaten verwendeten Flipwinkel, Echozeiten (TEᵢ) und/oder Wiederholzeiten (TRᵢ) derart optimiert wurden, dass die angeregten Spins in einem Pseudo Steady-State vorlagen.

7. Verfahren nach einem der vorhergehenden Ansprüche, wobei erfasste Bildpunkt-Zeit-Serien aus MR-Rohdaten erstellt werden, die mittels einer einzustrahlende RF-Pulse und zu schaltende Gradienten umfassenden Pulssequenz aufgenommen wurden, bei der die angewandten Wiederholzeiten (TRᵢ) so gewählt sind, dass Stoppbanden innerhalb eines durch die Pulssequenz angeregten Anregungsbereichs in dem Untersuchungsobjekt vermieden werden.

8. Verfahren nach Anspruch 7, wobei die Wiederholzeit TR derart gewählt wurde, dass ein Passband, insbesondere das zentrale Passband, der bei der Aufnahme der MR-Rohdaten verwendeten Pulssequenz breit genug ist, dass sowohl Spins einer ersten in dem Untersuchungsobjekt vorhandenen Spinspezies als auch Spins einer zweiten in dem Untersuchungsobjekt vorhandenen Spinspezies in dem Passband angeregt werden können, wobei die erste Spinspezies eine andere Resonanzfrequenz hat als die zweite Spinspezies.

9. Verfahren nach einem der vorhergehenden Ansprüche, wobei der Phasenzyklus der bei Aufnahme der MR-Rohdaten verwendeten RF-Pulse derart gewählt wurden, dass sowohl Spins einer ersten in dem Untersuchungsobjekt vorhandenen Spinspezies als auch Spins einer zweiten in dem Untersuchungsobjekt vorhandenen Spinspezies in einem Passband, insbesondere dem zentralen Passband, angeregt werden, wobei die erste Spinspezies eine andere Resonanzfrequenz hat als die zweite Spinspezies.

10. Verfahren nach einem der Ansprüche 1 bis 7, wobei Signale einer in dem Untersuchungsobjekt vorhandenen Spinspezies bei der Aufnahme von MR-Rohdaten unterdrückt wurden.

11. Verfahren nach Anspruch 10, wobei bei der Aufnahme von MR-Rohdaten zur Unterdrückung der Signale Binomialpulse verwendet werden.

12. Verfahren nach einem der vorhergehenden Ansprüche, wobei die Aufnahme der MR-Rohdaten zur Unterdrückung der Signale Präparationsblöcke zur Präparation einer gewünschten Magnetisierung umfasst, welche insbesondere periodisch in der Zeit angeordnet sind.

13. Magnetresonanzanlage (1) umfassend, eine Magneteinheit (3), eine Gradienteneinheit (5), eine Hochfrequenzeinheit (7) und eine Steuereinrichtung (9) mit einer Hochfrequenz-Sende-/Empfangs-Steuerung (7') und einer Parameterwertbestimmungseinheit (15), wobei die Steuereinrichtung (9) dazu ausgebildet ist, ein Verfahren nach einem der Ansprüche 1 bis 12 auf der Magnetresonanzanlage (1) auszuführen.

14. Computerprogramm, welches direkt in einen Speicher einer Steuereinrichtung (9) einer Magnetresonanzanlage (1) ladbar ist, mit Programm-Mitteln, um die Schritte des Verfahrens nach einem der Ansprüche 1 bis 12 auszuführen, wenn das Programm in der Steuereinrichtung (9) der Magnetresonanzanlage (1) ausgeführt wird.

15. Elektronisch lesbarer Datenträger mit darauf gespeicherten elektronisch lesbaren Steuerinformationen, welche zumindest ein Computerprogramm nach Anspruch 14 umfassen und derart ausgestaltet sind, dass sie bei Verwendung des Datenträgers in einer Steuereinrichtung (9) einer Magnetresonanzanlage (1) ein Verfahren nach einem der Ansprüche 1 bis 12 durchführen.
